# EUROPEAN PATENT SPECIFICATION

(11) **EP 3 237 372 B1**
(45) Date of publication and mention of the grant of the patent: **20.01.2021**
(21) Application number: 15831078.9
(22) Date of filing: 22.12.2015
(51) Int. Cl.: C07C 69/73, C07C 67/343, C07C 67/475

(54) **METHOD FOR PRODUCING OMEGA-HYDROXY FATTY ACID ESTER AND PRECURSOR COMPOUND THEREOF**
VERFAHREN ZUR HERSTELLUNG VON OMEGA-HYDROXY-FETTSÄUREESTER UND VORLÄUFERVERBINDUNG DAVON
PROCÉDÉ DE PRODUCTION D'ESTER D'ACIDE OMÉGA-HYDROXY GRAS ET SON COMPOSÉ PRÉCURSEUR

(30) Priority: 26.12.2014 JP 2014266683
(43) Date of publication of application: 01.11.2017
(73) Proprietor: Kao Corporation, Chuo-ku Tokyo 103-8210 (JP)
(72) Inventor: NAKAZAWA, Yuki, Wakayama 640-8580 (JP); AOKI, Takashi, Wakayama 640-8580 (JP)
(74) Representative: Hoffmann Eitle
(86) International application number: PCT/JP2015/006382
(87) International publication number: WO 2016/103677

(56) References cited:
- EP-A1- 0 908 455
- EP-A1- 2 210 870
- WO-A1-2015/136093
- DE-A1-102009 005 951
- US-A- 4 064 144
- VILLEMIN, DIDIER: "Synthesis of macrolides via metathesis", TETRAHEDRON LETTERS , 21(18), 1715-18 CODEN: TELEAY; ISSN: 0040-4039, 1980, XP055256705,
- WILLIAMS A S: "The Synthesis of Macrocyclic Musks", SYNTHESIS, GEORG THIEME VERLAG, STUTTGART, DE, no. 10, 1 January 1999 (1999-01-01), pages 1707-1723, XP002696528, ISSN: 0039-7881
- H. H. MATHUR ET AL: "658. Macrocyclic musk compounds. Part V. New syntheses of exaltone, exaltolide, dihydroambrettolide, and ?9-isoambrettolide from aleuritic acid", JOURNAL OF THE CHEMICAL SOCIETY (RESUMED), 1 January 1963 (1963-01-01), page 3505, XP055184965, ISSN: 0368-1769, DOI: 10.1039/jr9630003505
- FÜRSTNER A ET AL: "CONFORMATIONALLY UNBIASED MACROCYCLISATION REACTIONS BY RING CLOSING METATHESIS", THE JOURNAL OF ORGANIC CHEMISTRY, AMERICAN CHEMICAL SOCIETY, US, no. 61, 1 January 1996 (1996-01-01), pages 3942-3943, XP002077144, ISSN: 0022-3263, DOI: 10.1021/JO960733V

## Description

### FIELD OF THE INVENTION

The present invention relates to a method for producing an omega-hydroxy fatty acid ester and a precursor compound thereof represented by General Formula (III).

### BACKGROUND OF THE INVENTION

Many of macrocyclic lactones, particularly, macrocyclic lactones with a 14 or more-membered ring are important as fragrance materials with musk-like fragrance notes. Known examples of such macrocyclic lactones include cyclopentadecanolide, Ambrettolide, Habanolide, cyclohexadecanolide, and oxahexadecanolide ("Gosei Koryo, Kagaku to Shohin Chishiki" (Synthetic Perfumes, Chemistry and Commodity Knowledge), authored by Genichi Indo, Enlarged and Revised Edition, 2005, pp. 400-406).

A known method for producing these macrocyclic lactones is a ring-closure method in which an omega-hydroxy fatty acid ester is used as a raw material. For example, JP 2001-199976 A discloses a method for producing a macrocyclic lactone, in which an ester of hydroxy carboxylic acid with an alcohol having 13 to 40 carbon atoms is subjected to an intramolecular esterification reaction, for the purpose of producing it with high yield and high productivity.

On the other hand, in Tetrahedron Letters 1980, 21, 1715, (1) a method for synthesizing omega-hydroxy acid through an intermolecular metathesis reaction to give ring closure and (2) a method for synthesizing an ester having an unsaturated bond at each end to give ring closure through a metathesis reaction are described as the methods for synthesizing cyclopentadecanolide using a terminally unsaturated alcohol derivative and a terminally unsaturated carboxylic acid derivative as raw materials.

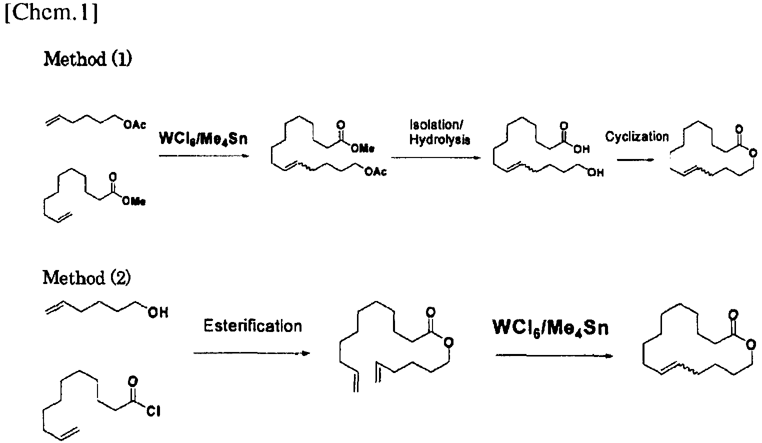

The use of a metathesis reaction makes it possible to relatively easily synthesize various macrocyclic compounds that differ from one another in the number of rings or the double bond position. JP 10(1998)-175882 A discloses a method for producing substituted and unsubstituted 12 to 21-membered lactones through a ring-closing metathesis reaction, wherein various esters having an unsaturated bond at each end are used as raw materials.

In a process for producing macrocyclic lactones through the ring-closing metathesis reaction, the reaction needs to be carried out usually under high dilution conditions (with a substrate concentration of 0.01 mol/L or lower) in order to prioritize the intramolecular reaction. Therefore, the process requires a large amount of reaction solvent and a reaction facility with an increased size, which has made it unsuitable for industrialization. On the other hand, high dilution conditions can be avoided when an omega-hydroxy fatty acid ester synthesized by the method of Tetrahedron Letters 1980, 21, 1715 is subjected to ring closure by the method of JP 2001-199976 A. However, the intermolecular metathesis reaction of Tetrahedron Letters 1980, 21, 1715 has problems in that the yield of the target omega-hydroxy fatty acid ester is low because of the production of unsaturated aliphatic diol and unsaturated aliphatic dicarboxylic acid due to dimerization of raw materials or the production of by-products due to, for example, isomerization of double bond positions.

WO 2015/136093 is a post-published document according to Article 54(3) EPC, which describes a method of forming a macrocyclic musk compound comprising the steps of:- i) cross-metathesizing a first olefin and a second olefin in the presence of a homogeneous transition metal catalyst comprising an alkylidene ligand, to form a statistical mixture of a hetero-dimer intermediate of said first and second terminal olefin, and homo-dimers ii) separating the hetero-dimer from the statistical mixture of hetero-and homo- dimers iii) and cyclizing the hetero-dimer intermediate to form the macrocyclic musk compound. EP 2 210 870 and DE 10 2009 005951 describe cross-metathesis reactions of olefinic compounds that contain at least one hydroxyl group and at least one carbon-carbon double bond, with at least one monounsaturated fatty acid or its derivative in the presence of a metathesis catalyst at at most 180°C.

### Summary of the Invention

The present inventors found that when a specific alcohol derivative and a specific carboxylic acid derivative are subjected to a metathesis reaction in the presence of a catalyst, a target omega-hydroxy fatty acid ester and a precursor thereof can be produced while the production of by-products due to, for example, dimerization of raw materials or isomerization of double bond positions is suppressed.

Therefore, with the foregoing in mind, it is an object of the present invention to provide a method for producing an omega-hydroxy fatty acid ester and a precursor compound thereof represented by Formula (III), comprising conducting a metathesis reaction of an alcohol derivative and a carboxylic acid derivative in the presence of a catalyst to provide a compound represented by Formula (III), wherein the alcohol derivative is a combination of a compound represented by Formula (VI) and a compound represented by Formula (I), and the carboxylic acid derivative is at least one selected from the group consisting of a compound represented by Formula (VII) and a compound represented by Formula (II):

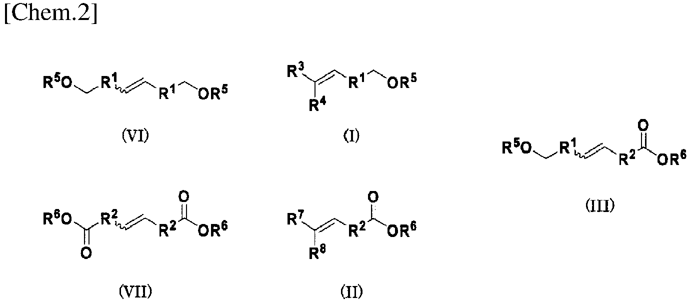

In the above formulae,
R¹ and R² are each independently a bond or a divalent hydrocarbon group having 1 to 14 carbon atoms that may have a substituent and may further contain an ether bond, a carbonyl bond, or both of them,
R³ and R⁴ are each independently a hydrogen atom or a linear alkyl group,
R⁵ is -C(=O)-R¹¹ (R¹¹ is an alkyl group having 1 to 4 carbon atoms),
R⁶ is an alkyl group having 1 to 4 carbon atoms, a benzyl group, or a phenethyl group, and
R⁷ and R⁸ are each independently a hydrogen atom or a linear alkyl group.

### DETAILED DESCRIPTION OF THE INVENTION

According to the present invention, compounds represented by Formula (III) can be produced while the production of by-products due to, for example, dimerization of raw materials or isomerization of double bond positions is suppressed.

In the present invention, the term "by-product" denotes a component that cannot be identified as any of the compounds of Formula (I), the compounds of Formula (II), the compounds of Formula (III), the compounds of Formula (VI), and the compounds of Formula (VII) when a reaction mixture is analyzed by the identification method described in the Example.

The present invention is a method for producing an omega-hydroxy fatty acid ester and a precursor compound thereof represented by Formula (III) in which an alcohol derivative and a carboxylic acid derivative are subjected to a metathesis reaction in the presence of a catalyst to obtain a compound represented by Formula (III) (hereinafter may also be referred to as a "compound (III)" or a "compound of Formula (III)"),
wherein the alcohol derivative is a combination of a compound represented by Formula (VI) (hereinafter may also be referred to as a "compound (VI)" or a "compound of Formula (VI)") and a compound represented by Formula (I) (hereinafter may also be referred to as a "compound (I)" or a "compound of Formula (I)"), and
the carboxylic acid derivative is at least one selected from the group consisting of a compound represented by Formula (VII) (hereinafter may also be referred to as a "compound (VII)" or a "compound of Formula (VII)") and a compound represented by Formula (II) (hereinafter may also be referred to as a "compound (II)" or a "compound of Formula (II)"),

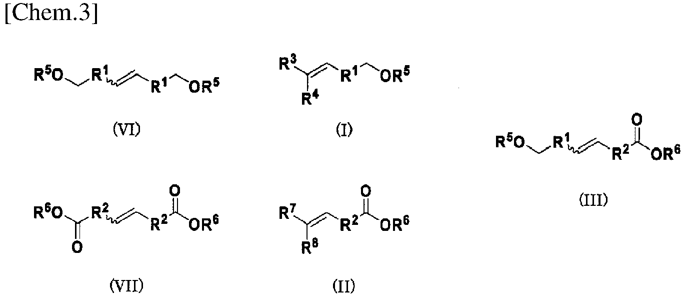

In the above formulae,
R¹ and R² are each independently a bond or a divalent hydrocarbon group having 1 to 14 carbon atoms that may have a substituent and may further contain an ether bond, a carbonyl bond, or both of them,
R³ and R⁴ are each independently a hydrogen atom or a linear alkyl group,
R⁵ is -C(=O)-R¹¹ (R¹¹ is an alkyl group having 1 to 4 carbon atoms),
R⁶ is an alkyl group having 1 to 4 carbon atoms, a benzyl group, or a phenethyl group, and
R⁷ and R⁸ are each independently a hydrogen atom or a linear alkyl group.

It is not clear why with a specific alcohol derivative and a specific carboxylic acid derivative subjected to a metathesis reaction in the presence of a catalyst, a compound of Formula (III), which is a target omega-hydroxy fatty acid ester or a precursor thereof, can be produced while the production of by-products due to, for example, dimerization of raw materials or isomerization of double bond positions is suppressed. However, it can be considered as follows.

It is considered that in the case where a compound of Formula (III), which is a omega-hydroxy fatty acid ester or a precursor thereof, is obtained by metathesis reaction, when a compound of Formula (I) and a compound of Formula (II) that are highly reactive and have little steric constraint are used as starting materials, a compound of Formula (VI) and a compound of Formula (VII), which are dimerization products of the raw materials, as well as by-products such as products produced due to isomerization of double bonds and reaction with the catalyst because of high reactivity tend to be produced in addition to a compound of Formula (III). In the present invention, it is considered that by using, as starting materials, a compound of Formula (VI), which is an internal olefinic alcohol derivative with a double bond located in the central part of the molecule and/or a compound of Formula (VII), which is an internal olefinic carboxylic acid derivative with a double bond located in the central part of the molecule, further dimerization of the raw materials can be suppressed, and side reactions such as isomerization can be suppressed due to the steric constraint in a state of interaction with the catalyst, and thereby a compound of Formula (III), which is a target cross-coupling compound, can be produced efficiently.

As described above, in the present invention,
the alcohol derivative is at least one selected from the group consisting of a compound of Formula (VI) and a compound of Formula (I), and
the carboxylic acid derivative is at least one selected from the group consisting of a compound of Formula (VII) and a compound of Formula (II).

That is, in the present invention, the alcohol derivative and the carboxylic acid derivative can be used as one of the combinations 6 - 8 shown in Table 1 below.

**[Table 1]**

| | Alcohol Derivative | | Carboxylic Acid Derivative | |
|---|---|---|---|---|
| Combination 1* | Compound of Formula (VI) | - | Compound of Formula (VII) | - |
| Combination 2* | Compound of Formula (VI) | - | - | Compound of Formula (II) |
| Combination 3* | - | Compound of Formula (I) | Compound of Formula (VII) | - |
| Combination 4* | Compound of Formula (VI) | - | Compound of Formula (VII) Formula | Compound of Formula (II) |
| Combination 5 | - | Compound of Formula (I) | Compound of Formula (VII) | Formula (II) Compound of Formula (II) |
| Combination 6 | Compound of Formula (VI) | Compound of Formula (I) | Compound of Formula (VII) | - |
| Combination 7 | Compound of Formula (VI) | Compound of Formula (I) | - | Compound of Formula (II) |
| Combination 8 | Compound of Formula (VI) | Compound of Formula (I) | Compound of Formula (VII) | Compound of Formula (II) |

| | | | | |
|---|---|---|---|---|
| * Reference Combinations | | | | |

In the present invention, a combination of a compound of Formula (VI) and a compound of Formula (1) is used as the alcohol derivative, since the production of by-products is suppressed.

In the present invention, it is preferable that a compound of Formula (VII) and a compound of Formula (II) be used as the carboxylic acid derivative, since the production of by-products is suppressed.

In the present invention, it is further preferable that the alcohol derivative be a compound of Formula (VI) and the carboxylic acid derivative be a compound of Formula (VII), since the production of by-products is suppressed.

In the present invention, the "hydrocarbon" in the "divalent hydrocarbon group having 1 to 14 carbon atoms" may be linear or may be branched. When the "divalent hydrocarbon group having 1 to 14 carbon atoms" is branched, the branch is preferably a methyl branch, a dimethyl branch, or an ethyl branch in terms of the fragrance note of macrocyclic lactone that is obtained by cyclization of a compound of Formula (III).

In the present invention, examples of the "substituent" in the "divalent hydrocarbon group having 1 to 14 carbon atoms that may have a substituent and may further contain an ether bond, a carbonyl bond, or both of them" include an alkoxy group, an alkylamino group, an alkoxycarbonyl group, an alkanoyl group, an aryl group, an aralkyl group, and an aryloxy group.

In the present invention, the "divalent hydrocarbon group having 1 to 14 carbon atoms that may further contain an ether bond, a carbonyl bond, or both of them" of the "divalent hydrocarbon group having 1 to 14 carbon atoms that may have a substituent and may further contain an ether bond, a carbonyl bond, or both of them" is, for example, a divalent hydrocarbon group having 1 to 14 carbon atoms that contains, in combination, at least one selected from the group consisting of an ether bond (-O-) and a carbonyl bond (-C(=O)-). However, the 1 to 14 carbon atoms do not include the carbon atom of the carbonyl bond. Examples of the "divalent hydrocarbon group having 1 to 14 carbon atoms that may have a substituent and may further contain an ether bond, a carbonyl bond, or both of them" include a formula -CH₂-O-CH₂-, a formula -CH₂-C(=O)-CH₂-, a formula -(CH₂)₂-O-CH₂-, a formula -(CH₂)₂-C(=O)-(CH₂) ₂-, a formula -(CH₂)₃-, a formula -(CH₂)₄-, a formula -(CH₂)₅-, a formula -(CH₂)₆-, a formula -(CH₂)₇-, and a formula -(CH₂)₈-, and the formula -(CH₂)₃-, the formula -(CH₂) ₅⁻, the formula -(CH₂)₇-, and the formula -(CH₂)₈- are preferred.

In the present invention, the "bond" represents a single bond between the atoms directly.

In the present invention, the "linear alkyl group" denotes, for example, a linear alkyl group having 1 to 10 carbon atoms, preferably a linear alkyl group having 1 to 6 carbon atoms, more preferably a linear alkyl group having 1 to 4 carbon atoms, and further preferably methyl or ethyl.

In the present invention, examples of the "alkyl group having 1 to 4 carbon atoms" include methyl, ethyl, n-propyl, i-propyl, n-butyl, i-butyl, sec-butyl, and tert-butyl. In terms of reactivity, it is preferably methyl or ethyl.

In the present invention, in the above formulae, preferably,
R¹ and R² are each independently a methylene group having 3 to 8 carbon atoms,
R³ and R⁴ are each independently a hydrogen atom,
R⁵ is -C(=O)-CH₃,
R⁶ is methyl, and
R⁷ and R⁸ are each independently a hydrogen atom.

In the present invention, in terms of improving the yield, it is preferable that the catalyst contain a metal selected from the group consisting of tungsten, molybdenum, rhenium, and ruthenium. Tungsten and ruthenium are more preferable. Particularly, examples of the catalyst include combinations of tungsten hexachloride and a cocatalyst, examples of which include alkyltins such as tetramethyltin and tetrabutyltin, alkylaluminiums such as triethylaluminum, alkylborons such as triethylborane, alkyl-lithiums such as n-butyllithium, and a hydride reagent such as NaBH₄, LiAlH₄, etc., as well as Umicore M (manufactured by Umicore Japan) and a Grubbs catalyst. Examples of Umicore M (manufactured by Umicore Japan) are shown below.

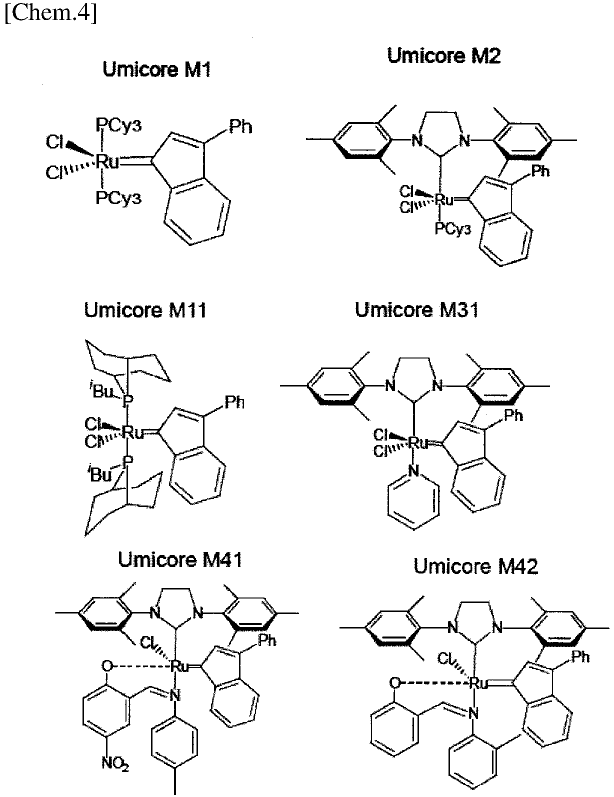

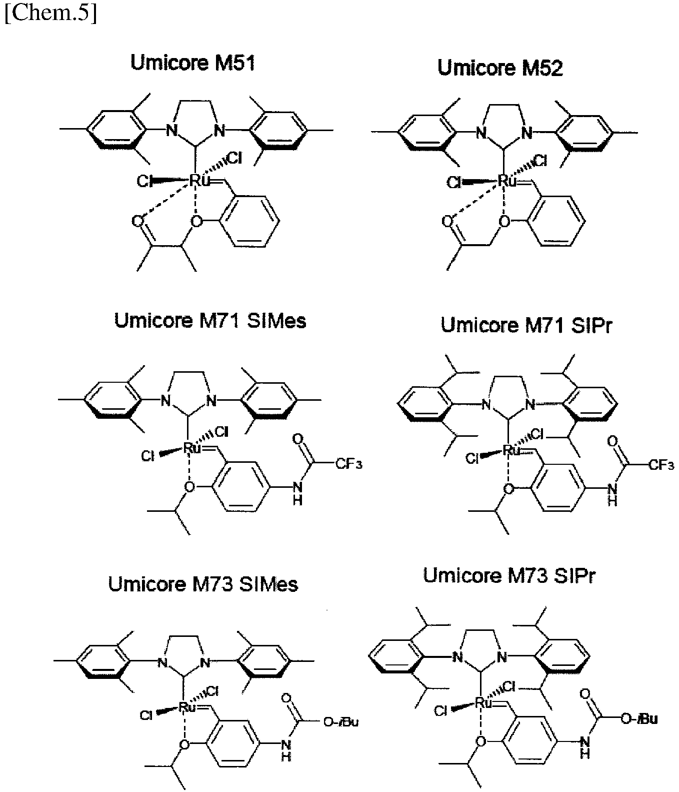

Examples of the Grubbs catalyst also are shown below.

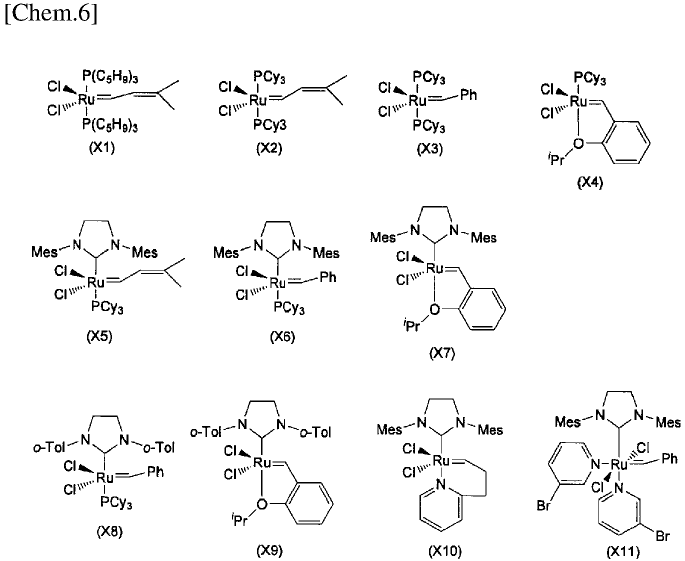

Among these, in terms of reactivity as well as in availability and economic terms, the catalyst is preferably a combination of tungsten hexachloride and tetramethyltin used as a cocatalyst, a combination of tungsten hexachloride and tetrabutyltin used as a cocatalyst, a combination of tungsten hexachloride and NaBH₄ used as a cocatalyst, or Umicore M, more preferably a combination of tungsten hexachloride and tetramethyltin used as a cocatalyst, a combination of tungsten hexachloride and tetrabutyltin used as a cocatalyst, or a combination of tungsten hexachloride and NaBH₄ used as a cocatalyst.

The amount of the catalyst to be used varies depending on the reactivity of the catalyst to be used. However, when a catalyst containing tungsten is used, it is preferably at least 1% by mole, more preferably at least 2.5% by mole with respect to the alcohol derivative, in terms of efficiently completing a reaction, and it is preferably not more than 20% by mole, more preferably not more than 10% by mole with respect to the alcohol derivative in economic terms and in terms of facilitating its removal after reaction.

When a catalyst containing ruthenium is used, it is preferably at least 0.1% by mole, more preferably at least 0.5% by mole with respect to the alcohol derivative in terms of efficiently completing a reaction, and it is preferably not more than 20% by mole, more preferably not more than 10% by mole, and further preferably not more than 5% by mole with respect to the alcohol derivative in economic terms and in terms of facilitating its removal after reaction.

When an alcohol derivative and a carboxylic acid derivative are subjected to a metathesis reaction in the presence of a catalyst, it is preferably that a solvent is used. Examples of the solvent include solvents inactive against the metathesis reaction, for example, a water-insoluble solvent. The water-insoluble solvent is preferably aliphatic hydrocarbon, aromatic hydrocarbon, halogenated aliphatic hydrocarbon, etc., and more preferably toluene or dichloromethane.

When an alcohol derivative and a carboxylic acid derivative are subjected to a metathesis reaction in the presence of a catalyst, it is preferable that the metathesis reaction is carried out under reflux with a solvent in terms of allowing the reaction to proceed efficiently.

When an alcohol derivative and a carboxylic acid derivative are subjected to a metathesis reaction in the presence of a catalyst, the reaction time is preferably at least four hours, more preferably at least five hours, and in terms of productivity, it is preferably not longer than 12 hours.

According to the method of the present invention, starting materials may remain together with a compound of Formula (III). In this case, the compound of Formula (III) may be isolated after the reaction, or the mixture thereof with the starting materials may be used for the subsequent reaction. However, in terms of simplifying the reaction, it is preferable that the compound of Formula (III) be isolated after the reaction to be used for the subsequent step.

Specifically, when a compound of Formula (VII) and a compound of Formula (I) are subjected to a metathesis reaction, the starting materials, the compound of Formula (VII) and the compound of Formula (I), as well as other compounds, a compound of Formula (VI) and a compound of Formula (II-1) may be obtained along with the compound of Formula (III). When the compound of Formula (III) is isolated and then the rest of the compounds are allowed to react by the method of the present invention again, the compound of Formula (III) further can be obtained.

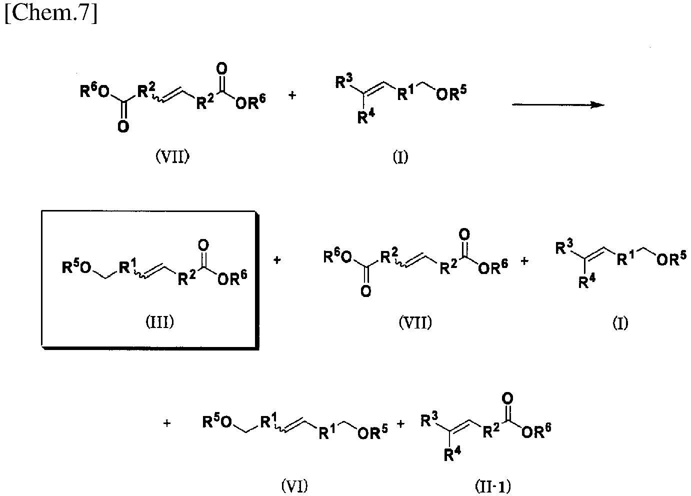

In the formulae above, the definitions of R¹, R², R³, R⁴, R⁵ and R⁶ are the same as those described above.

When the compound of Formula (III) is to be isolated after the reaction, it can be separated by rectification. With respect to the conditions for the rectification, the pressure is preferably 66 to 666 Pa (0.5 to 5 Torr), more preferably 66 to 133 Pa (0.5 to 1 Torr). The temperature for the rectification is preferably 100 to 350 degrees C, more preferably 150 to 300 degrees C.

### <Production of Macrocyclic Lactone>

With a compound of Formula (III) obtained according to the present invention being used as a starting material, macrocyclic lactone represented by Formula (IV) can be produced through (a) a cyclization step. Alternatively, with a compound of Formula (III) obtained according to the present invention being used as a starting material, macrocyclic lactone represented by Formula (V) can be produced through (a) the cyclization step and (b) a hydrogenation step. Alternatively, macrocyclic lactone represented by Formula (V) can also be produced by hydrogenating a compound of Formula (III) beforehand and then cyclizing it. The cyclization step and the hydrogenation step may be carried out, with the compound of Formula (III) obtained according to the present invention being contained in a mixture thereof with remaining compounds and compounds produced as by-products in the production method, or the cyclization step and the hydrogenation step may be carried out after the compound of Formula (III) is isolated and purified.

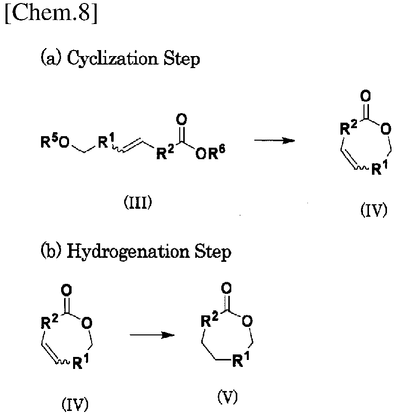

### (a) Cyclization Step

The cyclization step is a step for subjecting a compound of Formula (III) to an esterification reaction in an alcohol solvent (preferably a high-boiling alcohol to serve as a reaction accelerator).

The number of the carbon atoms of the alcohol that is used in this step is preferably at least 13, more preferably at least 18, and further preferably at least 20, and preferably not more than 40, more preferably not more than 36, and further preferably not more than 34. Examples of the alcohol include linear alcohols such as octadecanol, methyl branched alcohols such as 3,7,11,15-tetramethyl-2-hexadecenyl alcohol and 1-methyl pentadecanol, as well as Guerbet alkyl type branched alcohols such as 2-decyl-1-tetradecanol, 2-octyldodecanol, and 2-(1,3,3-trimethylbutyl)-5,7,7-trimethyloctanol. The above-mentioned alcohol is preferably a Guerbet alkyl type branched alcohol.

The cyclization step proceeds as follows. That is, with respect to a compound of Formula (III) in which R⁵ is -C(=O)-R¹¹ (R¹¹ is an alkyl group having 1 to 4 carbon atoms), transesterification thereof with a high-boiling alcohol results in an alcohol in which R⁵ in Formula (III) is a hydrogen atom, and the alcohol is intramolecularly esterified. In this step, polymers also are produced due to an intermolecular reaction as described below. In the following scheme, a compound of Formula (III-1) is shown, as an example, as a compound in which R⁵ in Formula (III) is Ac[-C(=O)-CH₃].

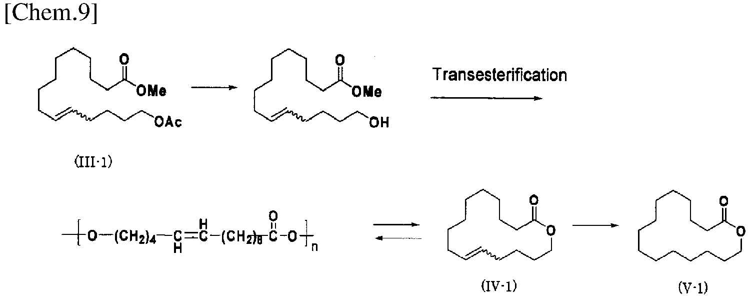

Under normal pressure, an intermolecular esterification reaction also proceeds to produce polymers. However, the resulting cyclized product (IV-1) is distilled out of the system under reduced pressure and thereby an equilibrium reaction can be biased toward the intramolecular cyclization reaction. In this case, since the compound produced by the transesterification, in which the high-boiling alcohol and R⁵ (Ac in the case of Formula (III-1)) have been bonded to each other, has a high boiling point, the compound is allowed to remain in the system without the distillation with the cyclized product (IV-1).

With respect to the amount of the alcohol (preferably a high-boiling alcohol), which is a reaction accelerator, in terms of yield and productivity, the molar ratio of [the alcohol] / [a carboxyl group and/or an ester group in the reaction mixture] is preferably at least 0.01, more preferably at least 0.1, and further preferably at least 0.3, and preferably not higher than 20, more preferably not higher than 10, further preferably not higher than 5, and still further preferably not higher than 2.

In this step, it is preferable to use a catalyst with an esterification or transesterification activity. Examples of the catalyst include metal oxide catalysts such as magnesium oxide, lead oxide, zirconium oxide, and zeolite, as well as neutral or alkaline catalysts including metal alkoxides such as titanium tetraisopropoxide, aluminum triisopropoxide, and sodium methoxide, metal hydroxides such as magnesium hydroxide, calcium hydroxide, sodium hydroxide, and potassium hydroxide, metal carbonate and metal salt of fatty acid such as magnesium carbonate, sodium carbonate, potassium carbonate, potassium acetate, calcium laurate, and sodium stearate, and others such as magnesium chloride. In terms of improving yield, catalysts containing a metal of group II, III or IV, such as magnesium oxide or titanium tetraisopropoxide are more preferred. The catalyst is further preferably titanium tetraisopropoxide in terms of lowering the reaction temperature.

The amount of the catalyst to be used in this step is, with respect to the compound of Formula (III), preferably at least 0.01% by mole, more preferably at least 0.1% by mole, and further preferably at least 0.3% by mole, and preferably not more than 50% by mole, more preferably not more than 20% by mole, and further preferably not more than 10% by mole.

In this step, the reaction pressure is preferably 66 to 666 Pa (0.5 to 5 Torr), more preferably 133 to 400 Pa (1 to 3 Torr).

In this step, the reaction temperature is preferably at least 100 degrees C, more preferably at least 120 degrees C, and further preferably at least 150 degrees C, and preferably not higher than 350 degrees C, more preferably not higher than 300 degrees C, and further preferably not higher than 250 degrees C.

This step carried out under such conditions allows water or a low molecular weight alcohol that is produced as a by-product to be removed, allows the esterification reaction to proceed, and allows a resulting unsaturated lactone of Formula (IV) to be obtained at high purity.

### (b) Hydrogenation Step

This step is a step for hydrogenating the unsaturated lactone of Formula (IV) obtained in the cyclization step to obtain a saturated lactone of Formula (V). The unsaturated lactone of Formula (IV) obtained in the cyclization step also is useful as a fragrance material with a musk-like odor. However, hydrogenating it results in an effect that the feeling of transparency and the feeling of cleanliness are further improved.

The conditions for the hydrogenation reaction are not particularly limited but it is preferable to use a hydrogenation catalyst. For the hydrogenation catalyst, any catalysts can be used as long as they have olefin hydrogenation activity but do not have ester-group hydrogenation activity. Particularly, a palladium catalyst, a ruthenium catalyst, and a platinum catalyst are preferable, and a palladium catalyst is more preferable.

In this step, the hydrogen pressure is preferably at least 0.01 MPa, more preferably at least 0.1 MPa, and preferably not higher than 10 MPa, more preferably not higher than 1 MPa. The temperature is preferably at least 20 degrees C and preferably not higher than 100 degrees C, more preferably not higher than 50 degrees C. It is further preferable to carry out this step at room temperature (25 degrees C).

It is preferable that the saturated lactone of Formula (V) obtained by the above-mentioned method be further subjected to rectification or silica gel column chromatography purification in terms of improving it to have a more preferable quality as a fragrance material with a musk-like odor.

With respect to the embodiment described above, the present invention further discloses a method for producing a compound of General Formula (III).
<1> A method for producing an omega-hydroxy fatty acid ester and a precursor compound thereof represented by Formula (III),
comprising conducting a metathesis reaction of an alcohol derivative and a carboxylic acid derivative in the presence of a catalyst to provide a compound represented by Formula (III),
wherein the alcohol derivative is a combination of a compound represented by Formula (VI) and a compound represented by Formula (I), and the carboxylic acid derivative is at least one selected from the group consisting of a compound represented by Formula (VII) and a compound represented by Formula (II), 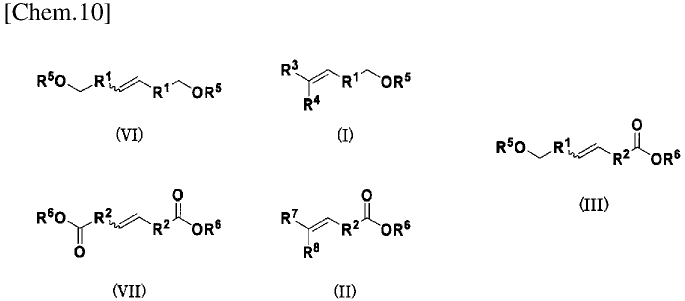
In the above formulae,
R¹ and R² are each independently a bond or a divalent hydrocarbon group having 1 to 14 carbon atoms that may have a substituent and may further contain an ether bond, a carbonyl bond, or both of them,
R³ and R⁴ are each independently a hydrogen atom or a linear alkyl group,
R⁵ is -C(=O)-R¹¹ (R¹¹ is an alkyl group having 1 to 4 carbon atoms),
R⁶ is an alkyl group having 1 to 4 carbon atoms, a benzyl group, or a phenethyl group, and
R⁷ and R⁸ are each independently a hydrogen atom or a linear alkyl group.

<2> The production method according to the item <1>, wherein the alcohol derivative and the carboxylic acid derivative can be used as any one of combinations 6 to 8 shown in Table 2 below.

**[Table 2]**

| | Alcohol Derivative | | Carboxylic Acid Derivative | |
|---|---|---|---|---|
| Combination 6 | Compound of Formula (VI) | Compound of Formula (I) | Compound of Formula (VII) | - |
| Combination 7 | Compound of Formula (VI) | Compound of Formula (I) | - | Compound of Formula (II) |
| Combination 8 | Compound of Formula (VI) | Compound of Formula (I) | Compound of Formula (VII) | Compound of Formula (II) |

<3> The production method according to the item <1> or <2>, wherein the alcohol derivative is a compound of Formula (VI) and a compound of Formula (I).
<4> The production method according to any one of the items <1> to <3>, wherein the carboxylic acid derivative is a compound of Formula (VII) and a compound of Formula (II).
<5> The production method according to the item <1> or <2>, wherein the alcohol derivative is a compound of Formula (VI), and the carboxylic acid derivative is a compound of Formula (VII).
<6> The production method according to any one of the items <1> to <5>, wherein the hydrocarbon of the divalent hydrocarbon group having 1 to 14 carbon atoms may be linear or may be branched, and when the divalent hydrocarbon group having 1 to 14 carbon atoms is branched, the branch is preferably a methyl branch, a dimethyl branch, or an ethyl branch.
<7> The production method according to any one of the items <1> to <6>, wherein the substituent is at least one selected from an alkoxy group, an alkylamino group, an alkoxycarbonyl group, an alkanoyl group, an aryl group, an aralkyl group, and an aryloxy group.
<8> The production method according to any one of the items <1> to <7>, wherein the divalent hydrocarbon group having 1 to 14 carbon atoms that may further contain an ether bond, a carbonyl bond, or both of them is a divalent hydrocarbon group having1 to 14 carbon atoms that contains a combination of at least one selected from the group consisting of an ether bond (-O-) and a carbonyl bond (-C(=O)-), and is preferably a formula -CH₂-O-CH₂-, a formula -CH₂-C(=O)-CH₂-, a formula -(CH₂)₂-O-CH₂-, a formula -(CH₂)₂-C(=O)-(CH₂)₂-, a formula -(CH₂)₃-, a formula -(CH₂)₄-, a formula -(CH₂)₅-, a formula -(CH₂)₆-, a formula -(CH₂)₇-, or a formula -(CH₂)₈-, more preferably a formula -(CH₂)₃-, a formula -(CH₂)₅-, a formula -(CH₂)₇-, or a formula - (CH₂)₈-.
<9> The production method according to any one of the items <1> to <8>, wherein the linear alkyl group is a linear alkyl group having 1 to 10 carbon atoms, preferably a linear alkyl group having 1 to 6 carbon atoms, more preferably a linear alkyl group having 1 to 4 carbon atoms, and further preferably methyl or ethyl.
<10> The production method according to any one of the items <1> to <9>, wherein the alkyl group having 1 to 4 carbon atoms is methyl, ethyl, n-propyl, i-propyl, n-butyl, i-butyl, sec-butyl, or tert-butyl, more preferably methyl or ethyl.
<11> The production method according to any one of the items <1> to <10>, wherein in the above formulae, preferably R¹ and R² are each independently a methylene group having 3 to 8 carbon atoms,
R³ and R⁴ are each independently a hydrogen atom,
R⁵ is -C(=O)-CH₃,
R⁶ is methyl, and
R⁷ and R⁸ are each independently a hydrogen atom.

<12> The production method according to any one of the items <1> to <11>, wherein the catalyst preferably contains a metal selected from the group consisting of tungsten, molybdenum, rhenium, and ruthenium, more preferably contains a metal selected from the group consisting of tungsten and ruthenium, and further preferably is selected from the group consisting of a combination of tungsten hexachloride and tetramethyltin used as a cocatalyst, a combination of tungsten hexachloride and tetrabutyltin used as a cocatalyst, a combination of tungsten hexachloride and NaBH₄ used as a cocatalyst, or Umicore M, more preferably a combination of tungsten hexachloride and tetramethyltin used as a cocatalyst, a combination of tungsten hexachloride and tetrabutyltin used as a cocatalyst, or a combination of tungsten hexachloride and NaBH 4 used as a cocatalyst.
<13> The production method according to the item <12>, wherein when the catalyst is a catalyst containing tungsten, the amount of the catalyst to be used is, with respect to the alcohol derivative, preferably at least 1% by mole, more preferably at least 2.5% by mole, and preferably not more than 20% by mole, more preferably not more than 10% by mole.
<14> The production method according to the item <12> or <13>, wherein when the catalyst is a catalyst containing ruthenium, the amount of the catalyst to be used is preferably at least 0.1% by mole, more preferably at least 0.5% by mole, and preferably not more than 20% by mole, more preferably not more than 10% by mole, and further preferably not more than 5% by mole.
<15> A method for producing a compound represented by Formula (IV), including a step of cyclizing a compound represented by Formula (III), wherein the compound represented by Formula (III) is obtained by a production method according to any one of the items <1> to <14>. 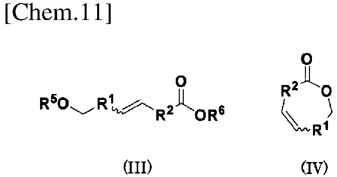
In the Formulae (III) and (IV),
R¹ and R² are each independently a bond or a divalent hydrocarbon group having 1 to 14 carbon atoms that may have a substituent and may further contain an ether bond, a carbonyl bond, or both of them,
R⁵ is -C(=O)-R¹¹ (R¹¹ is an alkyl group having 1 to 4 carbon atoms), and
R⁶ is an alkyl group having 1 to 4 carbon atoms, a benzyl group, or a phenethyl group.

<16> The production method according to the item <15>, wherein the step of cyclizing is a step of subjecting the compound of Formula (III) to an esterification reaction in an alcohol solvent (preferably a high-boiling alcohol to serve as a reaction accelerator).
<17> The production method according to the item <16>, wherein the number of the carbon atoms of the alcohol is preferably at least 13, more preferably at least 18, and further preferably at least 20, and preferably not more than 40, more preferably not more than 36, and further preferably not more than 34.
<18> The production method according to the item <16> or <17>, wherein the alcohol is preferably a linear alcohol (for example, octadecanol), a methyl branching type branched alcohol (for example, 3,7,11,15-tetramethyl-2-hexadecenyl alcohol and 1-methyl pentadecanol), or a Guerbet alkyl type branched alcohol (for example, 2-decyl-1-tetradecanol, 2-octyldodecanol, and 2-(1,3,3-trimethylbutyl)-5,7,7-trimethyloctanol), more preferably a Guerbet alkyl type branched alcohol.
<19> The production method according to any one of the items <16> to <18>, wherein with respect to the amount of the alcohol, the molar ratio of [the alcohol] / [a carboxyl group and/or an ester group in the reaction mixture] is preferably at least 0.01, more preferably at least 0.1, and further preferably at least 0.3, and preferably not higher than 20, more preferably not higher than 10, further preferably not higher than 5, and still further preferably not higher than 2.
<20> The production method according to any one of the items <15> to <19>, wherein in the step of cyclizing, it is preferable to use a catalyst with an esterification or transesterification activity, it is more preferable to use a catalyst containing a metal of group II, III, or IV, such as magnesium oxide or titanium tetraisopropoxide, and it is further preferable to use titanium tetraisopropoxide.
<21> The production method according to the item <20>, wherein the amount of the catalyst to be used is, with respect to the compound of Formula (III), preferably at least 0.01% by mole, more preferably at least 0.1% by mole, and further preferably at least 0.3% by mole, and preferably not more than 50% by mole, more preferably not more than 20% by mole, and further preferably not more than 10% by mole.
<22> A method for producing a compound represented by Formula (V), including a step of hydrogenating a compound represented by Formula (IV),
wherein the compound represented by Formula (IV) is obtained by a production method according to any one of the items <15> to <21>. 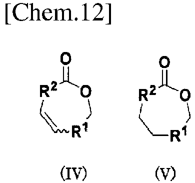
In the formulae (IV) and (V),
R¹ and R² are each independently a bond or a divalent hydrocarbon group having 1 to 14 carbon atoms that may have a substituent and may further contain an ether bond, a carbonyl bond, or both of them.
<23> The production method according to the item <22>, wherein in the step for hydrogenating, it is preferable to use a hydrogenation catalyst, it is more preferable to use a palladium catalyst, a ruthenium catalyst, or a platinum catalyst, and it is further preferable to use a palladium catalyst.

### Examples

In the following examples and comparative examples, the unit "%" denotes "% by mass" unless otherwise noted.

### <Reaction Yield>

The reaction yields indicated in the following examples were determined by the internal standard method of gas chromatography (GC) quantitative analysis.

### <Apparatus and Analytical Conditions for Gas Chromatography>

GC Apparatus: HP6850, manufactured by HEWLETT PACKARD
Column: DB-1 (with an inner diameter of 0.25 mm, a length of 30 m, and a film thickness of 0.25 µm), manufactured by J&W,
Carrier Gas: He, 1.5 mL/min
Injection Condition: 300 degrees C, Split Ratio: 1/100
Detection Condition: FID, 300 degrees C
Column Temperature Condition: 80 degrees C → Raised at 10 degrees C/min → Maintained at 300 degrees C for 10 minutes
Internal Standard Compound: n-Tridecane

### <Compound Identification>

Each compound obtained in the following examples and comparative examples was identified by spectrum analyses using a nuclear magnetic resonance spectrum (Mercury 400, manufactured by Varian) (¹H-NMR, ¹³C-NMR) and a gas chromatography mass spectrometer (GC-2010, manufactured by Shimadzu Corporation) (GC-MS).

### <Apparatus and Analytical Conditions for GC-MS>

GC-MS Apparatus: GC2010, manufactured by Shimadzu Corporation
Column: GLC LESTEK (with an inner diameter of 0.25 mm, a length of 25 m, and a film thickness of 0.25 µm), manufactured by Shimadzu Corporation
Carrier Gas: He, 1.82 mL/min
Injection Condition: 280 degrees C, Split Ratio: 1/50
Detector: GCMS-QP2010 Plus
Detection Temperature: 280 degrees C
Column Temperature Condition: 80 degrees C → Raised at 5 degrees C/min → Maintained at 280 degrees C for 10 minutes
Mass Range: Low: 70, High: 450

### Example 1 (Reference Example)

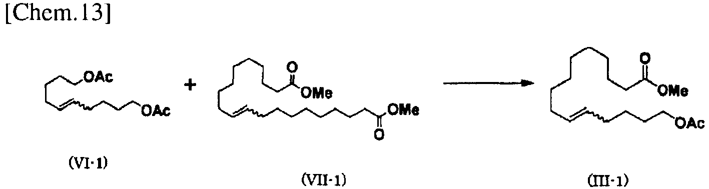

In a 200mL four-necked flask, to which a stirring rod, a condenser, a dropping funnel, and a thermometer were attached, 1 g (2.5 mmol) of tungsten hexachloride was placed and then the inside of the flask was subjected to nitrogen substitution. Toluene (5 mL) was added into the flask, and tetramethyltin (0.35 mL, 2.5 mmol) was dropped into the solution, which then was stirred at room temperature for 20 minutes.

Subsequently, to the reaction solution, 12.8 g of 5-decene-1,10-diacetate (VI-1) (with a purity of 100%, 50.1 mmol) and 19.0 g of dimethyl 10-eicosenedioate (VII-1) (with a purity of 97%, 50.0 mmol) were added through the dropping funnel. This then was allowed to react under a nitrogen gas stream at 110 degrees C for eight hours. Quantitativc analysis of the composition of the liquid obtained after completion of the reaction was performed by gas chromatography. As a result, it contained 7.5 g (24.0 mmol) of methyl 15-acetoxy-10-pentadecenoate (III-1), 9.2 g (35.9 mmol) of 5-decene-1,10-diacetate (VI-1), and 13.1 g (35.6 mmol) of dimethyl 10-eicosenedioate (VII-1). In this case, the GC area % of the by-products other than the above-mentioned compounds contained in the liquid obtained after completion of the reaction was 5.1%.

### Reference Examples 2 and 3 and Examples 4 and 5

These Examples were carried out in the same manner as Reference Example 1 except for using at least one selected from the group consisting of 5-decene-1,10-diacetate (VI-1) and 5-hexenyl acetate (I-1) instead of 5-decene-1,10-diacetate (VI-1) as well as at least one selected from the group consisting of dimethyl 10-eicosenedioate (VII-1) and methyl 10-undecylenate (II-1) instead of dimethyl 10-eicosenedioate (VII-1) in the amounts indicated in Table 3. Table 3 shows the types and amounts (obtained by GC analysis) of the compounds thus obtained.

### Comparative Example1

Comparative Example 1 was carried out in the same manner as in Example 1 except for using 5-hexenyl acetate (1-1) instead of 5-decene-1,10-diacetate (VI-1) and methyl 10-undecylenate (II-1) instead of dimethyl 10-cicoscnedioate (VII-1) in the amounts indicated in Table 3. Table 3 shows the types and amounts (obtained by GC analysis) of the compounds thus obtained.

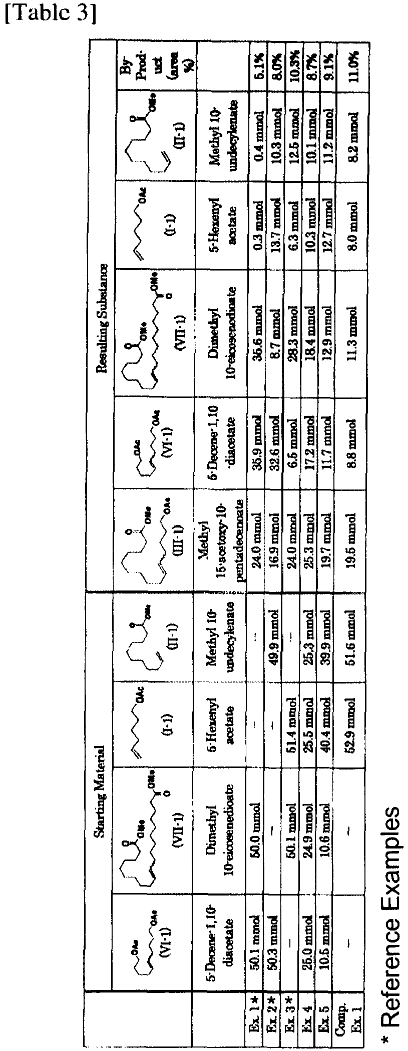

As shown in Table 3 above, according to the production method of the present invention, the compound of Formula (III) was able to be produced while the production of by-products due to, for example, dimerization of the raw materials or isomerization of double bond positions was suppressed.

### Example 6 (Reference Example)

Reference Example 6 was carried out in the same manner as Reference Example 1 except for using 7-tetradecene-1,14-diacetate (VI-2) instead of 5-decene-1,10-diacetate (VI-1) and dimethyl 9-octadecenedioate (VII-2) instead of dimethyl 10-eicosenedioate (VII-1) in the amounts indicated in Table 4. Table 4 shows the types and amounts (obtained by GC analysis) of the compounds thus obtained.

### Comparative Example2

Comparative Example 2 was carried out in the same manner as in Example 1 except for using 7-octenyl acetate (1-2) instead of 5-decene-1,10-diacetate (VI-1) and methyl 9-decenoate (II-2) instead of dimethyl 10-eicosenedioate (VII-1) in the amounts indicated in Table 4. Table 4 shows the types and amounts (obtained by GC analysis) of the compounds thus obtained.

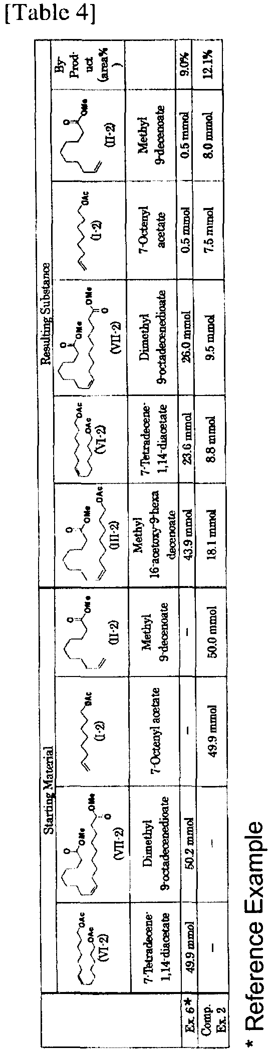

As shown in Table 4 above, according to the production method of the present invention, the compound of Formula (III) was able to be produced while the production of by-products due to, for example, dimerization of the raw materials or isomerization of double bond positions was suppressed.

### Example 7 (Reference Example)

In a 50mL two-necked flask, to which a condenser was attached, 526.0 mg of 5-decene-1,10-diacetate (VI-1) (with a purity of 99%, 2.0 mmol) and 738.1 mg of dimethyl 10-eicosenedioate (VII-1) (with a purity of 100%, 2.0 mmol) were placed and then were dissolved in 5 mL of dichloromethane. Subsequently, 55 mg (0.06 mmol) of metathesis catalyst Umicore M1 (manufactured by Umicore Japan, obtained from Wako Pure Chemical Industries, Ltd.) was added thereto, which then was heated to reflux under a nitrogen gas stream for ten hours. Quantitative analysis of the composition of the liquid obtained after completion of the reaction was performed by gas chromatography. As a result, it contained 589.0 mg (1.9 mmol) of methyl 15-acetoxy-10-pentadecenoate (III-1), 249.2 mg (0.97 mmol) of 5-decene-1,10-diacetate (VI-1), and 382.7 mg (1.0 mmol) of dimethyl 10-eicosencdioate (VII-1). In this case, the CG area % of the by-products other than the above-mentioned compounds contained in the liquid obtained after completion of the reaction was 2.3%.

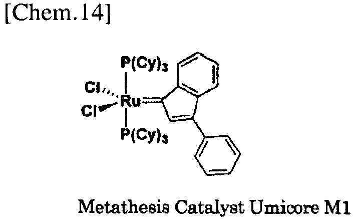

### Comparative Example 3

Comparative Example 3 was carried out in the same manner as in Example 7 except for using 100 mg (0.11 mmol) of metathesis catalyst Umicore M1 (manufactured by Umicore Japan, obtained from Wako Pure Chemical Industries, Ltd.) instead of 55 mg (0.06 mmol) of metathesis catalyst Umicore M1 (manufactured by Umicore Japan, obtained from Wako Pure Chemical Industries, Ltd.), 9 mL of dichloromethane instead of 5 mL of dichloromethane as well as 5-hexenyl acetate (1-1) instead of 5-decene-1,10-diacetate (VI-1) and methyl 10-undecylenate (II-1) instead of dimethyl 10-eicosenedioate (VII-1) in the amounts indicated in Table 5. Table 5 shows the types and amounts (obtained by GC analysis) of the compounds thus obtained.

### Example 8 (Reference Example)

Reference Example 8 was carried out in the same manner as Reference Example 7 except for using 8.8 mg (9.3 × 10⁻³ mmol) of metathesis catalyst Umicore M2 (manufactured by Umicore Japan, obtained from Wako Pure Chemical Industries, Ltd.) instead of 55 mg (0.06 mmol) of metathesis catalyst Umicore M1 (manufactured by Umicore Japan, obtained from Wako Pure Chemical Industries, Ltd.) and using 5-decene-1,10-diacetate (VI-1) and dimethyl 10-eicosenedioate (VII-1) in the amounts indicated in Table 5. Table 5 shows the types and amounts (obtained by GC analysis) of the compounds thus obtained.

### Comparative Example 4

Comparative Example 4 was carried out in the same manner as in Comparative Example 3 except for using 17.5 mg (18.4 × 10⁻³ mmol) of metathesis catalyst Umicore M2 (manufactured by Umicore Japan, obtained from Wako Pure Chemical Industries, Ltd.) instead of 55 mg (0.06 mmol) of metathesis catalyst Umicore M1 (manufactured by Umicore Japan, obtained from Wako Pure Chemical Industries, Ltd.) and10 mL of dichloromethane instead of 9 mL of dichloromethane. Table 5 shows the types and amounts (obtained by GC analysis) of the compounds thus obtained.

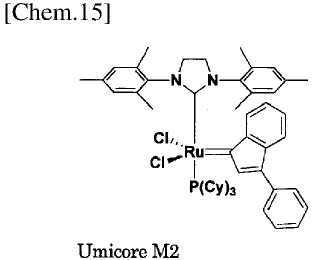

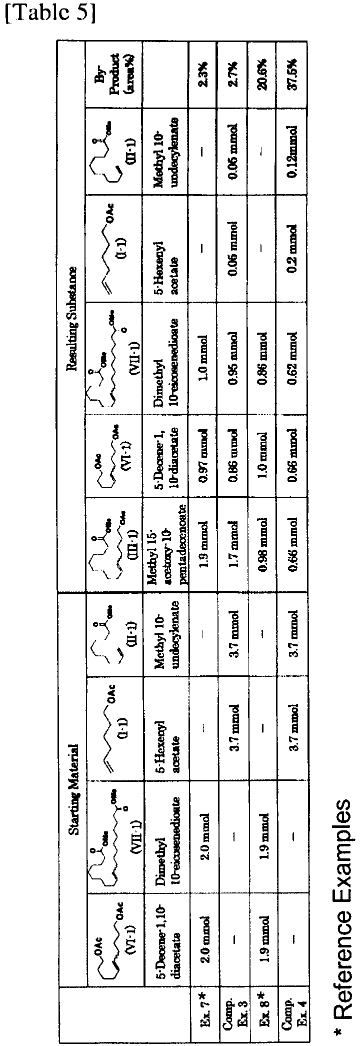

As shown in Table 5, according to the production method of the present invention, the compound of Formula (III) was able to be produced while the production of by-products due to, for example, dimerization of the raw materials or isomerization of double bond positions was suppressed.

### Example 9 (Reference Example)

### Synthesis of Macrocyclic Lactone

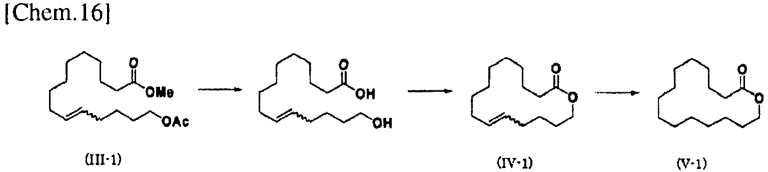

The liquid obtained after completion of the metathesis reaction in Ref. Ex. 1 was washed with an aqueous NaOH solution. Thereafter, the layers were separated and the organic layer was extracted. The organic layer was washed with saturated brine and then sodium sulfate was added to dry the organic layer. After the solid was removed by membrane filtration, the solvent was evaporated using a rotary evaporator. The reaction mixture thus obtained was distilled under reduced pressure to be purified and thereby the colorless viscous fraction that was distilled at 170 to 175 degrees C/133 Pa, i.e., methyl 15-acetoxy-10-pentadecenoate (III-1) was obtained in a purity of 95.3%.

In a 300mL four-necked flask, to which a Claisen distillation head and a thermometer were attached, 87.7 g of methyl 15-acetoxy-10-pentadecenoate (III-1) (with a purity of 95.3%, 267.4 mmol), 105.3 g of 2-decyl-1-tetradecanol (296.9 mmol, the molar ratio of [the high-boiling alcohol] / [the carboxyl group and/or the ester group in the reaction mixture] = 0.56), and 0.88 g of titanium tetraisopropoxide (3.1 mmol, 1.04% by mole with respect to the compound of Formula (III-1)) were placed. The temperature thereof was raised to 170 degrees C at normal pressure under a nitrogen gas stream. This was stirred for two hours while methanol was distilled. Subsequently, the reaction was allowed to proceed while oxacyclohexadec-11-en-2-one (IV-1), which was produced under the conditions of 220 to 250 degrees C and 133 to 65 Pa (1.0 to 0.5 Torr), was distilled, and thereby 161.0 g of colorless liquid fraction was obtained. The fraction was subjected to gas chromatography quantitative analysis. As a result, it contained 59.5 g of oxacyclohexadec-1 1-en-2-one (IV-1) (249.8 mmol, yield 93.4%). The fraction obtained above was distilled under reduced pressure to be purified, and thereby a colorless viscous fraction that was distilled at 120-125 degrees C/133 Pa, i.e., oxacyclohexadec-11-en-2-one (IV-1) (with a purity of 100%) was isolated.

In a 100mL two-necked flask, to which a pressure-resistant balloon filled with hydrogen gas was attached, 392.4 mg of oxacyclohexadec-11-en-2-one (IV-1) (with a purity of 100%, 1.65 mmol) and 8.0 mg of Pd/C catalyst (5% Wet) were placed and then were dissolved in 10 mL of methanol. The inside of the flask was subjected to pressure reduction with a vacuum line and then to hydrogen substitution. This was stirred at room temperature (25 degrees C) for 24 hours. The liquid obtained after completion of the reaction was subjected to gas chromatography quantitative analysis. As a result, it contained 388.7 mg of oxacyclohexadecan-2-one (V-1) (1.62 mmol, yield 98.2%).

### Example 10 (Reference Example)

### Synthesis of Macrocyclic Lactone

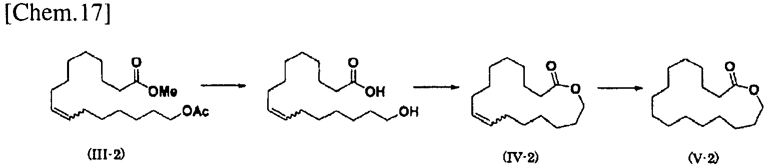

The liquid obtained after completion of the metathesis reaction in Ref. Ex. 6 was washed with an aqueous NaOH solution. Thereafter, the layers were separated and the organic layer was extracted. The organic layer was washed with saturated brine and then sodium sulfate was added to dry the organic layer. After the solid was removed by membrane filtration, the solvent was evaporated using a rotary evaporator. The reaction mixture thus obtained was distilled under reduced pressure to be purified and thereby the colorless viscous fraction that was distilled at 177-179 degrees C/133Pa, i.e., methyl 16-acetoxy-9-hexadecenoate was obtained in a purity of 91.2%.

In a 300mL four-necked flask, to which a Claisen distillation head and a thermometer were attached, 58.0 g of methyl 16-acetoxy-9-hexadecenoate (III-2) (with a purity of 91.2%, 161.9 mmol), 73.4 g of 2-decyl-1-tecradecanol (206.8 mmol, the molar ratio of [the high-boiling alcohol] / [the carboxyl group and/or the ester group in the reaction mixture] = 0.64), and 0.64 g of titanium tetraisopropoxide (2.2 mmol, 1.36% by mole with respect to the compound of Formula (III-2)) were placed. The temperature thereof was raised to 170 degrees C at normal pressure under a nitrogen gas stream. This was stirred for two hours while methanol was distilled. Subsequently, the reaction was allowed to proceed while oxacycloheptadec-10-en-2-one (IV-2), which was produced under the conditions of 220 to 250 degrees C and 133 to 65 Pa (1.0 to 0.5 Torr), was distilled, and thereby 110.8 g of colorless liquid fraction was obtained. The fraction was subjected to gas chromatography quantitative analysis. As a result, it contained 39.8 g of oxacycloheptadec-10-en-2-one (IV-2) (157.8 mmol, yield 97.5%). The fraction obtained above was distilled under reduced pressure to be purified, and thereby a colorless viscous fraction that was distilled at 143 to 147 degrees C/133 Pa, i.e., oxacycloheptadec- 10-en-2-one (IV-2) (with a purity of 100%) was isolated.

In a 50mL two-necked flask, to which a pressure-resistant balloon filled with hydrogen gas was attached, 1.0 g of oxacycloheptadec-10-en-2-one (IV-2) (with a purity of 100%, 4.0 mmol) and 20 mg of Pd/C catalyst (5% W et) were placed and then were dissolved in 10 mL of methanol. The inside of the flask was subjected to pressure reduction with a vacuum line and then to hydrogen substitution. This was stirred at room temperature (25 degrees C) for three hours. The liquid obtained after completion of the reaction was subjected to gas chromatography quantitative analysis. As a result, it contained 0.99 g of oxacycloheptadecan-2-one (V-2) (3.9 mmol, yield 99.0%).

According to the production method of the present invention, the compound of Formula (III) can be produced while the production of by-products due to, for example, dimerization of raw materials or isomerization of double bond positions is suppressed. The production method of the present invention provides an effect that the compound of Formula (VI) and the compound of Formula (VII), which are by-products other than the compound of Formula (III) obtained by the metathesis reactions of the compound of Formula (I) and the compound of Formula (II), can be used effectively as starting materials for obtaining the compound of Formula (III).

## Claims

1. A method for producing an omega-hydroxy fatty acid ester and a precursor compound thereof represented by Formula (III), comprising conducting a metathesis reaction of an alcohol derivative and a carboxylic acid derivative in the presence of a catalyst to provide a compound represented by Formula (III),
wherein the alcohol derivative is a combination of a compound represented by Formula (VI) and a compound represented by Formula (I), and
the carboxylic acid derivative is at least one selected from the group consisting of a compound represented by Formula (VII) and a compound represented by Formula (II), 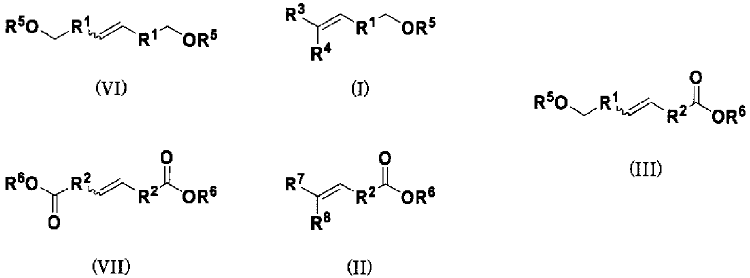
wherein R¹ and R² are each independently a bond or a divalent hydrocarbon group having 1 to 14 carbon atoms that may have a substituent and may further contain an ether bond, a carbonyl bond, or both of them,
R³ and R⁴ are each independently a hydrogen atom or a linear alkyl group,
R⁵ is -C(=O)-R¹¹ (R¹¹ is an alkyl group having 1 to 4 carbon atoms),
R⁶ is an alkyl group having 1 to 4 carbon atoms, a benzyl group, or a phenethyl group, and
R⁷ and R⁸ are each independently a hydrogen atom or a linear alkyl group.

2. The production method according to claim 1, wherein the carboxylic acid derivative is a compound represented by Formula (VII) and a compound represented by Formula (II).

3. The production method according to claim 1, wherein the alcohol derivative is a compound represented by Formula (VI), and
the carboxylic acid derivative is a compound represented by Formula (VII).

4. The production method according to any one of claims 1 to 3, wherein the catalyst contains a metal selected from the group consisting of tungsten, molybdenum, rhenium, and ruthenium.

5. The production method according to any one of claims 1 to 4, wherein the divalent hydrocarbon group having 1 to 14 carbon atoms that may further contain an ether bond, a carbonyl bond, or both of them is a divalent hydrocarbon group having 1 to 14 carbon atoms that comprises, in combination, at least one selected from the group consisting of an ether bond (-O-) and a carbonyl bond (-C(=O)-).

6. The production method according to claim 5, wherein the divalent hydrocarbon group having 1 to 14 carbon atoms that may further contain an ether bond, a carbonyl bond, or both of them is a formula -CH₂-O-CH₂-, a formula -CH₂-C(=O)-CH₂-, a formula -(CH₂)₂-O-CH₂-, a formula -(CH₂)₂-C(=O)-(CH₂)₂-, a formula -(CH₂)₃-, a formula -(CH₂)₄-, a formula -(CH₂)₅-, a formula -(CH₂)₆-, a formula -(CH₂)₇-, or a formula -(CH₂)₈-.

7. The production method according to any one of claims 1 to 6, wherein the linear alkyl group is a linear alkyl group having 1 to 4 carbon atoms.

8. The production method according to any one of claims 1 to 7, wherein
R¹ and R² are each independently a methylene group having 3 to 8 carbon atoms,
R³ and R⁴ are each independently a hydrogen atom,
R⁵ is -C(=O)-CH₃,
R⁶ is methyl, and
R⁷ and R⁸ are each independently a hydrogen atom.

9. The production method according to any one of claims 1 to 8, wherein
the catalyst is selected from a group consisting of
a combination of tungsten hexachloride and tetramethyltin used as a cocatalyst, a combination of tungsten hexachloride and tetrabutyltin used as a cocatalyst, a combination of tungsten hexachloride and NaBH₄ used as a cocatalyst, and Umicore M.

10. The production method according to any one of claims 1 to 9, wherein
the catalyst is selected from a group consisting of
a combination of tungsten hexachloride and tetramethyltin used as a cocatalyst, a combination of tungsten hexachloride and tetrabutyltin used as a cocatalyst, and a combination of tungsten hexachloride and NaBH₄ used as a cocatalyst.

11. The production method according to any one of claims 1 to 10, wherein
when the catalyst comprises tungsten is used, the amount of the catalyst to be used is at least 1% by mole and not more than 20% by mole with respect to the alcohol derivative.

12. A method for producing a compound represented by Formula (IV), comprising a step of obtaining a compound represented by Formula (III) by the production method according to any one of claims 1 to 11, and a step of cyclizing the obtained compound represented by Formula (III), 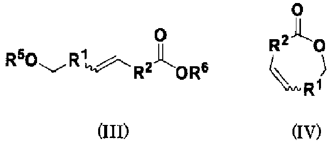
wherein R¹ and R² are each independently a bond or a divalent hydrocarbon group having 1 to 14 carbon atoms that may have a substituent and may further contain an ether bond, a carbonyl bond, or both of them,
R⁵ is -C(=O)-R¹¹ (R¹¹ is an alkyl group having 1 to 4 carbon atoms), and
R⁶ is an alkyl group having 1 to 4 carbon atoms, a benzyl group, or a phenethyl group.

13. The production method according to claim 12, wherein
the cyclization step is a step for subjecting a compound of Formula (III) to an esterification reaction in an alcohol solvent.

14. A method for producing a compound represented by Formula (V), comprising a step of obtaining a compound represented by Formula (III) as defined in claim 1 by the production method according to any one of claims 1 to 11, a step of cyclizing the obtained compound of formula (III) and hydrogenating a compound represented by Formula (IV), wherein the compound represented by Formula (IV) is obtained by a production method according to claim 12 or 13, 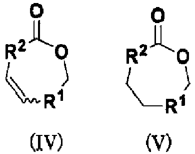 wherein R¹ and R² are each independently-a bond or a divalent hydrocarbon group having 1 to 14 carbon atoms that may have a substituent and may further contain an ether bond, a carbonyl bond, or both of them.

15. The production method according to claim 14, in a step of hydrogenating a compound represented by Formula (IV), a palladium catalyst, a ruthenium catalyst, or a platinum catalyst is used.

## Patentansprüche

1. Verfahren zur Erzeugung eines Omega-Hydroxy-Fettsäureesters und einer Vorläuferverbindung davon, dargestellt durch die Formel (III), enthaltend die Durchführung einer Metathese-Reaktion eines Alkohol-Derivates und eines Carbonsäure-Derivates in der Gegenwart eines Katalysators, unter Erhalt einer Verbindung mit der Formel (III),
worin das Alkoholderivat eine Kombination aus einer Verbindung mit der Formel (VI) und einer Verbindung mit der Formel (I) ist und
das Carbonsäurederivat zumindest eines ist, ausgewählt aus der Gruppe, bestehend aus einer Verbindung mit der Formel (VII) und eine Verbindung mit der Formel (II) 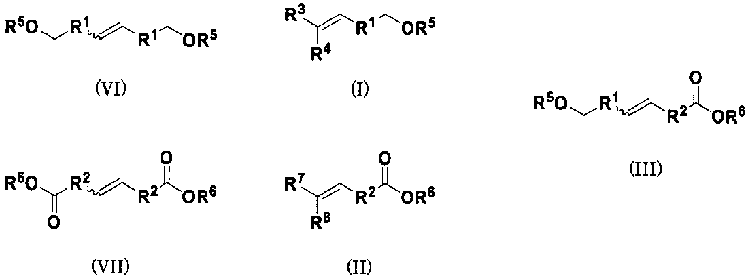
worin R¹ und R² jeweils unabhängig eine Bindung oder bivalente Kohlenwasserstoffgruppe mit 1 bis 14 Kohlenstoffatomen sind, die einen Substituenten haben können und weiterhin eine Etherbindung, Carbonylbindung oder beides aufweisen können,
R³ und R⁴ jeweils unabhängig ein Wasserstoffatom oder eine lineare Alkylgruppe sind,
R⁵ -C(=O)-R¹¹ ist (R¹¹ ist eine Alkylgruppe mit 1 bis 4 Kohlenstoffatomen),
R⁶ eine Alkylgruppe mit 1 bis 4 Kohlenstoffatomen, Benzylgruppe oder Phenethylgruppe ist und
R⁷ und R⁸ jeweils unabhängig ein Wasserstoffatom oder lineare Alkylgruppe sind.

2. Produktionsverfahren gemäß Anspruch 1, worin das Carbonsäurederivat eine Verbindung mit der Formel (VII) und eine Verbindung mit der Formel (II) ist.

3. Produktionsverfahren gemäß Anspruch 1, worin das Alkoholderivat eine Verbindung mit der Formel (VI) ist und das Carbonsäurederivat eine Verbindung mit der Formel (VII) ist.

4. Produktionsverfahren gemäß einem der Ansprüche 1 bis 3, worin der Katalysator ein Metall enthält, ausgewählt aus der Gruppe, bestehend aus Wolfram, Molybdän, Rhenium und Ruthenium.

5. Produktionsverfahren gemäß einem der Ansprüche 1 bis 4, worin die bivalente Kohlenwasserstoffgruppe mit 1 bis 14 Kohlenstoffatomen, die weiterhin eine Etherbindung, Carbonylbindung oder beides enthält, eine bivalente Kohlenwasserstoffgruppe mit 1 bis 14 Kohlenstoffatomen ist, die in Kombination zumindest eines enthält, ausgewählt aus der Gruppe, bestehend aus einer Etherbindung (-O-) und einer Carbonylbindung (-C(=O)-).

6. Produktionsverfahren gemäß Anspruch 5, worin die bivalente Kohlenwasserstoffgruppe mit 1 bis 14 Kohlenstoffatomen, die weiterhin eine Etherbindung, Carbonylbindung oder beides haben kann, eine Formel -CH₂-O-CH₂-, Formel -CH₂-C(=O)-CH₂-, Formel -(CH₂)₂-O-CH₂⁻, Formel -(CH₂)₂-C(=O)-(CH₂)₂-, Formel -(CH₂)₃-, Formel -(CH₂)₄-, Formel -(CH₂)₅-, Formel -(CH₂)₆-, Formel -(CH₂)₇- oder
Formel -(CH₂)₈- ist.

7. Produktionsverfahren gemäß einem der Ansprüche 1 bis 6, worin die lineare Alkylgruppe eine lineare Alkylgruppe mit 1 bis 4 Kohlenstoffatomen ist.

8. Produktionsverfahren gemäß einem der Ansprüche 1 bis 7, worin R¹ und R² jeweils unabhängig eine Methylengruppe mit 3 bis 8 Kohlenstoffatomen sind,
R³ und R⁴ jeweils unabhängig ein Wasserstoffatom sind,
R⁵ -C(=O)-CH₃ ist, R⁶ Methyl ist und
R⁷ und R⁸ jeweils unabhängig ein Wasserstoffatom sind.

9. Produktionsverfahren gemäß einem der Ansprüche 1 bis 8, worin der Katalysator ausgewählt ist aus einer Gruppe, bestehend aus einer Kombination von Wolframhexachlorid und Tetramethylzinn, das als Co-Katalysator verwendet wird,
einer Kombination von Wolframhexachlorid und Tetrabutylzinn, das als Co-Katalysator verwendet wird,
einer Kombination von Wolframhexachlorid und NaBH₄, das als Co-Katalysator verwendet wird, und Umicore M.

10. Produktionsverfahren gemäß einem der Ansprüche 1 bis 9, worin der Katalysator aus einer Gruppe ausgewählt ist, bestehend aus einer Kombination aus Wolframhexachlorid und Tetramethylzinn, das als Co-Katalysator verwendet wird, einer Kombination aus Wolframhexachlorid und Tetrabutylzinn, das als Co-Katalysator verwendet wird, und einer Kombination aus Wolframhexachlorid und NaBH₄, das als Co-Katalysator verwendet wird.

11. Produktionsverfahren gemäß einem der Ansprüche 1 bis 10, worin dann, wenn der Katalysator verwendet wird, der Wolfram enthält, die Menge des zu verwendenden Katalysators zumindest 1 mol-% und nicht mehr als 20 mol-% in Bezug auf das Alkoholderivat ist.

12. Verfahren zur Erzeugung einer Verbindung mit der Formel (IV), enthaltend einen Schritt für den Erhalt einer Verbindung mit der Formel (III) durch das Produktionsverfahren gemäß einem der Ansprüche 1 bis 11 und einen Schritt zum Zyklisieren der erhaltenen Verbindung mit der Formel (III) 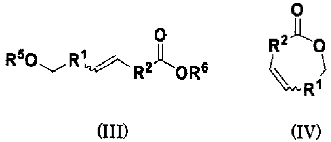
worin R¹ und R² jeweils unabhängig eine Bindung oder eine bivalente Kohlenwasserstoffgruppe mit 1 bis 14 Kohlenstoffatomen sind, die einen Substituenten haben können und weiterhin eine Etherbindung, Carbonylbindung oder beides aufweisen können,
R⁵ C(=O)-R¹¹ ist (R¹¹ ist eine Alkylgruppe mit 1 bis 4 Kohlenstoffatomen) und
R⁶ eine Alkylgruppe mit 1 bis 4 Kohlenstoffatomen, Benzylgruppe oder Phenethylgruppe ist.

13. Produktionsverfahren gemäß Anspruch 12, worin der Zyklisierungsschritt ein Schritt zum Durchführen einer Veresterungsreaktion mit einer Verbindung der Formel (III) in einem Alkohollösungsmittel ist.

14. Verfahren zur Erzeugung einer Verbindung mit der Formel (V), enthaltend einen Schritt zum Erhalt einer Verbindung mit der Formel (III), wie in Anspruch 1 definiert, durch das Produktionsverfahren gemäß einem der Ansprüche 1 bis 11, einen Schritt zum Zyklisieren der erhaltenen Verbindung mit der Formel (III) und Hydrieren einer Verbindung mit der Formel (IV),
worin die Verbindung mit der Formel (IV) durch ein Produktionsverfahren gemäß Anspruch 12 oder 13 erhalten ist, 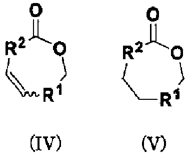 worin R¹ und R² jeweils unabhängig eine Bindung oder bivalente Kohlenwasserstoffgruppe mit 1 bis 14 Kohlenstoffatomen sind, die einen Substituenten und weiterhin eine Etherbindung, Carbonylbindung oder beides enthalten können.

15. Produktionsverfahren gemäß Anspruch 14, worin in einem Schritt zur Hydrierung einer Verbindung mit der Formel (IV) ein Palladiumkatalysator, Ruthenkatalysator oder Platinkatalysator verwendet wird.

## Revendications

1. Procédé de production d'un ester d'oméga-hydroxy acide gras et d'un composé précurseur de celui-ci représenté par la formule (III), comprenant la mise en œuvre d'une réaction de métathèse d'un dérivé d'alcool et d'un dérivé d'acide carboxylique en présence d'un catalyseur pour fournir un composé représenté par la formule (III),
dans lequel le dérivé d'alcool est une combinaison d'un composé représenté par la formule (VI) et d'un composé représenté par la formule (I), et
le dérivé d'acide carboxylique est au moins un sélectionné dans le groupe consistant en un composé représenté par la formule (VII) et un composé représenté par la formule (II), 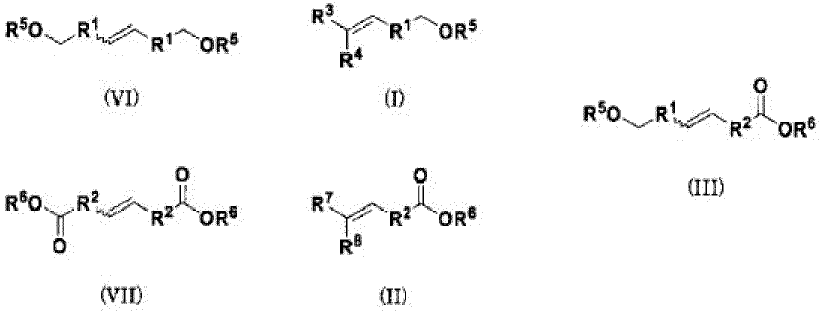
dans lesquelles R¹ et R² sont chacun indépendamment une liaison ou un groupement hydrocarboné divalent présentant 1 à 14 atomes de carbone qui peut présenter un substituant et peut contenir en outre une liaison éther, une liaison carbonyle, ou les deux,
R³ et R⁴ sont chacun indépendamment un atome d'hydrogène ou un groupement alkyle linéaire,
R⁵ est -C(=O)-R¹¹ (R¹¹ est un groupement alkyle présentant 1 à 4 atomes de carbone),
R⁶ est un groupement alkyle présentant 1 à 4 atomes de carbone, un groupement benzyle, ou un groupement phénéthyle, et
R⁷ et R⁸ sont chacun indépendamment un atome d'hydrogène ou un groupement alkyle linéaire.

2. Procédé de production selon la revendication 1, dans lequel le dérivé d'acide carboxylique est un composé représenté par la formule (VII) et un composé représenté par la formule (II).

3. Procédé de production selon la revendication 1, dans lequel le dérivé d'alcool est un composé représenté par la formule (VI), et
le dérivé d'acide carboxylique est un composé représenté par la formule (VII).

4. Procédé de production selon l'une quelconque des revendications 1 à 3, dans lequel le catalyseur contient un métal sélectionné dans le groupe consistant en le tungstène, le molybdène, le rhénium, et le ruthénium.

5. Procédé de production selon l'une quelconque des revendications 1 à 4, dans lequel le groupement hydrocarboné divalent présentant 1 à 14 atomes de carbone qui peut contenir en outre une liaison éther, une liaison carbonyle, ou les deux est un groupement hydrocarboné divalent présentant 1 à 14 atomes de carbone qui comprend, en combinaison, au moins un sélectionné dans le groupe consistant en une liaison éther (-O-) et une liaison carbonyle (-C(=O)-).

6. Procédé de production selon la revendication 5, dans lequel le groupement hydrocarboné divalent présentant 1 à 14 atomes de carbone qui peut contenir en outre une liaison éther, une liaison carbonyle, ou les deux est une formule -CH₂-O-CH₂-, une formule -CH₂-C(=O)-CH₂-, une formule-(CH₂)₂-O-CH₂-, une formule-(CH₂)₂-C(=O)-(CH₂)₂-, une formule-(CH₂)₃-, une formule-(CH₂)₄-, une formule-(CH₂)₅-, une formule-(CH₂)₆-, une formule-(CH₂)₇-, ou une formule-(CH₂)₈-.

7. Procédé de production selon l'une quelconque des revendications 1 à 6, dans lequel le groupement alkyle linéaire est un groupement alkyle linéaire présentant 1 à 4 atomes de carbone.

8. Procédé de production selon l'une quelconque des revendications 1 à 7, dans lequel
R¹ et R² sont chacun indépendamment un groupement méthylène présentant 3 à 8 atomes de carbone,
R³ et R⁴ sont chacun indépendamment un atome d'hydrogène,
R⁵ est -C(=O)-CH₃,
R⁶ est un méthyle, et
R⁷ et R⁸ sont chacun indépendamment un atome d'hydrogène.

9. Procédé de production selon l'une quelconque des revendications 1 à 8, dans lequel
le catalyseur est sélectionné dans un groupe consistant en
une combinaison d'hexachlorure de tungstène et de tétraméthylétain utilisé comme cocatalyseur,
une combinaison d'hexachlorure de tungstène et de tétrabutylétain utilisé comme cocatalyseur,
une combinaison d'hexachlorure de tungstène et de NaBH₄ utilisé comme cocatalyseur, et
l'Umicore M.

10. Procédé de production selon l'une quelconque des revendications 1 à 9, dans lequel
le catalyseur est sélectionné dans un groupe consistant en
une combinaison d'hexachlorure de tungstène et de tétraméthylétain utilisé comme cocatalyseur,
une combinaison d'hexachlorure de tungstène et de tétrabutylétain utilisé comme cocatalyseur, et
une combinaison d'hexachlorure de tungstène et de NaBH₄ utilisé comme cocatalyseur.

11. Procédé de production selon l'une quelconque des revendications 1 à 10, dans lequel
quand le catalyseur comprend du tungstène est utilisé, la quantité du catalyseur à utiliser est au moins de 1 % en mole et pas supérieure à 20 % en mole par rapport au dérivé d'alcool.

12. Procédé de production d'un composé représenté par la formule (IV), comprenant une étape d'obtention d'un composé représenté par la formule (III) par le procédé de production selon l'une quelconque des revendications 1 à 11, et une étape de cyclisation du composé obtenu représenté par la formule (III), 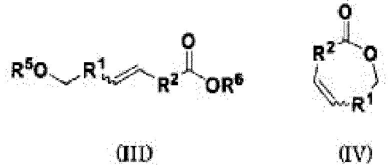
dans lesquelles R¹ et R² sont chacun indépendamment une liaison ou un groupement hydrocarboné divalent présentant 1 à 14 atomes de carbone qui peut présenter un substituant et peut contenir en outre une liaison éther, une liaison carbonyle, ou les deux,
R⁵ est -C(=O)-R¹¹ (R¹¹ est un groupement alkyle présentant 1 à 4 atomes de carbone), et
R⁶ est un groupement alkyle présentant 1 à 4 atomes de carbone, un groupement benzyle, ou un groupement phénéthyle.

13. Procédé de production selon la revendication 12, dans lequel
l'étape de cyclisation est une étape pour soumettre un composé de formule (III) à une réaction d'estérification dans un solvant de type alcool.

14. Procédé de production d'un composé représenté par la formule (V), comprenant une étape d'obtention d'un composé représenté par la formule (III) telle que définie dans la revendication 1 par le procédé de production selon l'une quelconque des revendications 1 à 11, un étape de cyclisation du composé obtenu de formule (III) et
l'hydrogénation d'un composé représenté par la formule (IV),
dans lequel le composé représenté par la formule (IV) est obtenu par un procédé de production selon la revendication 12 ou 13, 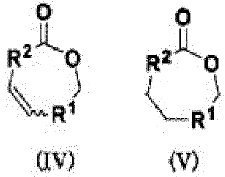 dans lesquelles R¹ et R² sont chacun indépendamment une liaison ou un groupement hydrocarboné divalent présentant 1 à 14 atomes de carbone qui peut présenter un substituant et peut contenir en outre une liaison éther, une liaison carbonyle, ou les deux.

15. Procédé de production selon la revendication 14, dans une étape d'hydrogénation d'un composé représenté par la formule (IV), un catalyseur au palladium, un catalyseur au ruthénium, ou un catalyseur au platine est utilisé.
